# EUROPEAN PATENT APPLICATION

(11) **EP 4 461 297 A1**
(43) Date of publication of application: **13.11.2024**
(21) Application number: 23753110.8
(22) Date of filing: 07.02.2023
(51) Int. Cl.: A61K 31/5377, A61P 3/04, A61K 9/00

(54) **USE OF MELANOCORTIN-4 RECEPTOR AGONIST IN PREVENTING OR TREATING RARE GENETIC OBESITY DISEASE**

(30) Priority: 08.02.2022 KR 20220016377
(71) Applicant: LG Chem, Ltd., Yeongdeungpo-gu Seoul 07336 (KR)
(72) Inventor: PARK, Hee Dong, Seoul 07796 (KR); HWANG, Hye Kyeong, Seoul 07796 (KR); YEO, Su Jin, Seoul 07796 (KR)
(74) Representative: Hoffmann Eitle
(86) International application number: PCT/KR2023/001712
(87) International publication number: WO 2023/153762

(57) **Abstract**

The preset invention relates to a use of a compound of chemical formula 1 or a pharmaceutically acceptable salt thereof for preventing or treating rare genetic obesity diseases associated with a damaged melanocortin-4 receptor (MC4R) pathway, particularly rare genetic obesity diseases associated with proopiomelanocortin (POMC) deficiency.

## Description

### Technical Field

The present invention relates to the use of a compound of the Formula 1 or a pharmaceutically acceptable salt thereof for the purpose of preventing or treating a rare genetic obesity disease associated with the melanocortin-4 receptor (MC4R) pathway, particularly a rare genetic obesity disease associated with proopiomelanocortin (POMC) deficiency:

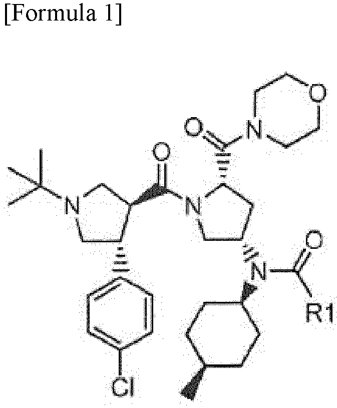

In the Formula 1, R1 is C₂-C₅ alkyl.

### Background Technology

Melanocortin receptor (MCR) is a type of G-protein-coupled receptor (GPCR), and the main role of G-protein is to activate secondary messengers to regulate the cellular response to many physiological stimuli through signal transduction. Five types of melanocortin receptors have been identified to date. MC1R is expressed in melanocytes and macrophages, and in melanocytes, it determines the color of skin and hair by regulating the melanin pigment. MC2R is expressed in the adrenal gland and adipose tissue, and its mediating function in the regulation of adrenal hormone secretion by adrenocorticotropic hormone in the adrenal gland is well known. MC3R, MC4R, and MC5R are expressed in the brain as well as nerve terminals and are thought to mediate central neuronal actions of melanocortin peptides, which manifest as effects on behavior, learning, memory, appetite, and neuronal development and regeneration. To date, MC3R is known to be involved in erectile dysfunction and inflammatory responses, and MC4R is known to be involved in obesity and diabetes, and research on the specificity of each receptor is actively being conducted (MacNeil DJ et al., Eur J Pharmacol 2002, 450, 93). As a result, genetic studies in humans with active obesity have shown that MC4R is deeply involved, and genetically modified mice (knockout mice) in which MC4R is removed have been shown to develop obesity due to overeating, proving that this receptor plays an important role in appetite regulation ((Lu D, Willard D et al., Nature 1994, 371(6500), 799; Huszar D et al., Cell 1997, 88(1), 131; Hinney A et al., J Clin Endocrinol Metab 1990, 84(4), 1483).

On the other hand, the existing obesity treatments are mainly appetite suppressants that act on the central nervous system, and most of them are drugs that regulate the action of neurotransmitters (e.g., phentermine, mazindol, lorcaserin, fluoxetine, and sibutramine). However, these neurotransmitter modulators exert a wide range of effects on various physiological functions other than appetite suppression through numerous subtype receptors. Therefore, in the case of above drugs, there is a disadvantage in that they lack selectivity for each subtype, and thus, when administered over a long period of time, they cause various side effects. On the other hand, melanocortin antagonists, which are neuropeptides rather than neurotransmitters, have the advantage of being able to induce weight loss through appetite suppression without affecting other physiological functions, given that all other functions other than energy metabolism are normal in MC4R gene knockout (KO) mice.

### Detailed Description of the Invention

### Problem to be Solved

The present invention is intended to provide a compound of the Formula 1 or a pharmaceutically acceptable salt thereof, for use in the prevention or treatment of rare genetic obesity disease associated with the melanocortin-4 receptor (MC4R) pathway, particularly rare genetic obesity disease associated with proopiomelanocortin (POMC) deficiency:

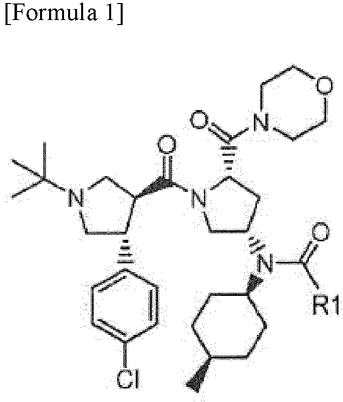

In the Formula 1, R1 is C₂-C₅ alkyl.

### Means of Solving the Problem

The present invention provides a medicament for the preventing or treating a rare genetic obesity disease associated with proopiomelanocortin (POMC) deficiency, comprising a therapeutically effective amount of a compound of the Formula 1 or a pharmaceutically acceptable salt thereof:

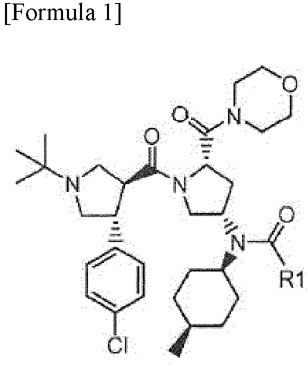

In the Formula 1, R1 is C₂-C₅ alkyl.

The present invention also provides pharmaceutical composition for the preventing or treating a rare genetic obesity disease associated with proopiomelanocortin (POMC) deficiency, comprising a therapeutically effective amount of a compound of the Formula 1 or a pharmaceutically acceptable salt thereof, together with a pharmaceutically acceptable carrier.

In addition, the present invention provides a method of preventing or treating a rare genetic obesity disease associated with proopiomelanocortin (POMC) deficiency, comprising administering to a subject in need thereof a therapeutically effective amount of a compound of the Formula 1 or a pharmaceutically acceptable salt thereof.

The present invention also provides a use of a compound of the Formula 1 or a pharmaceutically acceptable salt thereof, for preventing or treating a rare genetic obesity disease associated with proopiomelanocortin (POMC) deficiency.

Hereinafter, the present invention will be described in more detail.

According to one aspect of the present invention, there is provided a medicament for preventing or treating rare genetic obesity associated with a proopiomelanocortin (POMC) deficiency, comprising a therapeutically effective amount of a compound of Formula 1 or a pharmaceutically acceptable salt thereof.

According to another aspect of the present invention, there is provided a pharmaceutical composition for preventing or treating rare genetic obesity associated with proopiomelanocortin (POMC) deficiency, comprising a therapeutically effective amount of a compound of Formula 1 or a pharmaceutically acceptable salt thereof, together with a pharmaceutically acceptable carrier.

In one embodiment according to the present invention, the compound of Formula 1 is *N-*((3*S*,5*S*)-1-((3*S*,4*R*)-1-(*tert*-butyl)-4-(4-chlorophenyl)pyrrolidin-3-carbonyl)-5-(morpholine-4-carbonyl)pyrrolidin-3-yl)-*N*-((1*s*,4*R*)-4-methylcyclohexyl)isobutyramide of Formula 2 below:

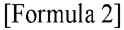

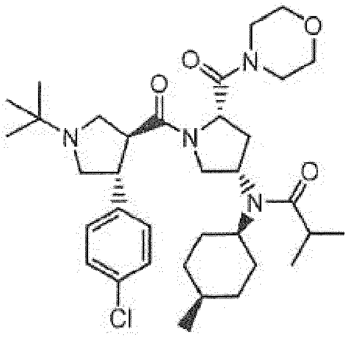

In another embodiment according to the present invention, the pharmaceutically acceptable salts include, but are not limited to, an acid addition salt formed by an inorganic acid such as hydrochloric acid, sulfuric acid, nitric acid, phosphoric acid, hydrobromic acid, hydroiodic acid; an organic carboxylic acid such as tartaric acid, formic acid, citric acid, acetic acid, trichloroacetic acid, trifluoroacetic acid, gluconic acid, benzoic acid, lactic acid, fumaric acid, maleic acid, and the like; and a sulfonic acid such as methanesulfonic acid, benzenesulfonic acid, p-toluenesulfonic acid, or naphthalenesulfonic acid, and the like. In other embodiments according to the present invention, the pharmaceutically acceptable salts may be selected from the group consisting of hydrochloric acid, sulfuric acid, nitric acid, phosphoric acid, hydrobromic acid, and hydroiodic acid. In an embodiment according to the present invention, the pharmaceutically acceptable salt is a hydrochloride salt.

In another embodiment according to the present invention, the hydrochloride salt of the compound of the Formula 1 can be prepared according to Scheme 1 below. However, a person of ordinary skill in the art pertaining to the present invention can prepare the compound of Formula 1 by various methods based on the structure of Formula 1.

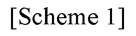

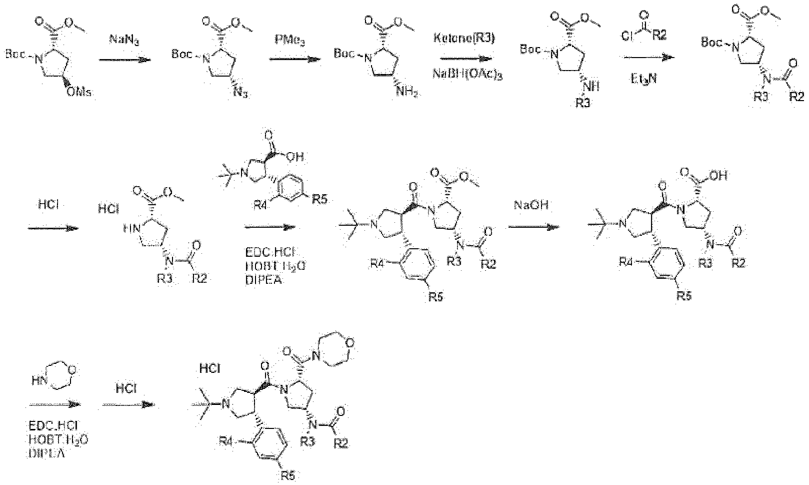

In the above Scheme 1,
R2 is C₁-C₅ alkyl;
R3 is C₃-C₈ cycloalkyl which is unsubstituted or substituted with one or two C₁-C₅ alkyl; and
R4 and R5 are each independently hydrogen or halogen.

According to the present invention, the compound of Formula 1 can efficiently prevent or treat rare genetic obesity disease associated with proopiomelanocortin (POMC) deficiency. Proopiomelanocortin (POMC) is a precursor polypeptide with 241 amino acid residues, which is synthesized in the anterior and intermediate pituitary gland and acts as a precursor of several peptide hormones such as melanocyte-stimulating hormone (MSH), endorphin, and corticotrophin. As a ligand of the melanocortin-4 receptor (MC4R), alpha-MSH acts upstream of the MC4R and can cause severe early-onset obesity if the Pomc gene encoding it is deficient and not expressed normally.

In another embodiment according to the present invention, a "therapeutically effective amount" for an individual subject means an amount sufficient to achieve the pharmacological effect described above, i.e., a therapeutic effect, and the amount of the compound will depend on the condition of the subject and its severity, the method of administration, and the age of the subject to be treated, but can be determined by a person of ordinary skill in the relevant art based on their own knowledge.

In another embodiment according to the present invention, the therapeutically effective dosage of the compound of Formula 1, for example, the dosage required to treat an adult, is typically in the range of about 0.1 to 500 mg per day, depending on the frequency and intensity of administration. For intramuscular or intravenous administration to an adult, a total dosage of about 0.1 to 300 mg per day, divided into single doses, will usually be sufficient, although a higher daily dosage may be desirable for some patients.

In the present invention, a "pharmaceutical composition" may include, in addition to the active compound according to the present invention, other chemical components such as carriers, diluents, excipients, and the like. Thus, the pharmaceutical composition may include pharmaceutically acceptable carriers, diluents, excipients, or combinations thereof, as needed. The pharmaceutical composition facilitates the administration of the active compound into an organism. Various techniques for administering compounds exist, including, but not limited to, oral, injection, aerosol, parenteral, and topical administration.

As used herein, a "carrier" means a compound that facilitates the introduction of a compound into a cell or tissue. For example, dimethyl sulfoxide (DMSO) is a common carrier that facilitates the introduction of many organic compounds into cells or tissues of an organism.

As used herein, a "diluent" is defined as a compound that is diluted in water that stabilizes the biologically active form of the subject compound, as well as dissolves the compound. Salts dissolved in buffers are used as diluents in the art. A commonly used buffer is phosphate-buffered saline, which mimics the salt form of human body fluids. Because buffer salts can control the pH of a solution at low concentrations, it is rare for buffered diluents to alter the biological activity of a compound.

As used herein, "pharmaceutically acceptable" means properties that do not impair the biological activity and physical properties of a compound.

In the present invention, the compound can be formulated into various pharmaceutical dosage forms, depending on the purpose. When preparing a pharmaceutical composition according to the present invention, an active ingredient, specifically the compound of Formula 1 or a pharmaceutically acceptable salt thereof, is mixed with a variety of pharmaceutically acceptable carriers that may be selected depending on the formulation to be prepared. For example, the pharmaceutical composition according to the present invention can be formulated as an injectable formulation, an oral formulation, or the like, depending on the purpose.

The compound of the present invention may be formulated by a known method using known pharmaceutical carriers and excipients and may be introduced in a unit dose form or in a multidose container. The form of the formulation may be in the form of a solution, suspension or emulsion in an oil or aqueous medium and may contain conventional dispersants, suspending agents or stabilizing agents. It may also be in the form of a dry powder, for example, dissolved in sterile, pyrogen-free water prior to use. The compound of the present invention may also be formulated in a suppository form using a conventional suppository base such as cocoa butter or other glycerides. Solid dosage forms for oral administration can be capsules, tablets, pills, granules, and tablets, with capsules and tablets being particularly useful. Tablets and pills are preferably prepared with enteric coatings. Solid dosage forms can be prepared by mixing the compound of the present invention with one or more inert diluents, such as sucrose, lactose, starch, and the like, and carriers, such as lubricants, disintegrants, binders, and the like, such as magnesium stearate. It can also be formulated in a transdermal dosage form, for example, as a lotion, ointment, gel, cream, patch or spray. In another embodiment according to the present invention, the medicament or pharmaceutical composition may be an oral formulation.

As used herein, "prevention" means reducing or eliminating the likelihood of contracting a disease.

As used herein, "treatment" means stopping, delaying, or alleviating the progression of a disease when used on a subject exhibiting symptoms of the disease.

### Effect of the Invention

The medicament or pharmaceutical composition according to the present invention can efficiently prevent or treat rare genetic obesity diseases associated with the melanocortin-4 receptor (MC4R) pathway, in particular rare genetic obesity diseases associated with proopiomelanocortin (POMC) deficiency.

### Brief Description of the Drawings

FIG. 1 is a graph showing the results of anti-obesity efficacy in a high fat diet (HFD) POMC heterozygous deficient mouse model.
FIG. 2 is a graph showing the results of anti-obesity efficacy in a high fat diet (HFD) POMC homozygous deficient mouse model.

### Best Form for Carrying Out the Invention

Hereinafter, the present invention will be described in more detail through working examples. However, these working examples are intended to illustrate one or more embodiments by way of example only, and the scope of the invention is not limited thereby.

### Example of Preparation: Synthesis of N-((3S,5S)-1-((3S,4R)-1-(tert-butyl)-4-4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

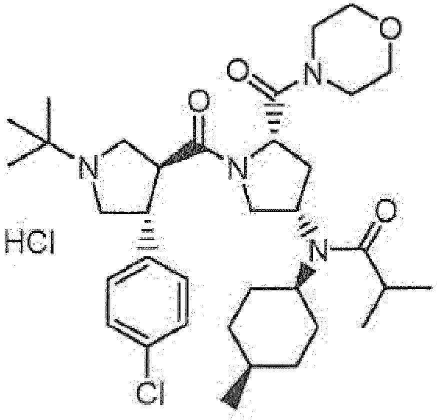

The title compound was obtained through the following steps A, B, C and D.

### Step A: Preparation of methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidin-2-carboxylate

Methyl (2*S*,4*S*)-4-(*N*-((1*S*,4*R*)-4-methylcyclohexyl)isobutyramido)pyrrolidine-2-carboxylate hydrochloride obtained from Example 1 of preparation (28.7 g, 82.73 mmol), (3*S*,4*R*)-1-(*tert-*butyl)-4-(4-chlorophenyl)pyrrolidine-3-carboxylic acid (24.5 g, 86. 87 mmol), 1-(3-dimethylaminopropyl)-3-ethylcarbodiimide hydrochloride (22.2 g, 115.83 mmol) and 1-hydroxybenzotriazole hydrate (15.7 g, 115.83 mmol) from Example 2 of preparation were dissolved in *N*,*N'*-dimethylformamide (400 ml) and *N*,*N*'-diisopropylethylamine (72.0 ml, 413.66 mmol) was added slowly. After stirring at room temperature for 16 hours and the reaction solvent was concentrated under reduced pressure, 0.5 N aqueous sodium hydroxide solution was added and extracted twice with ethyl acetate. The organic layer was washed twice with aqueous sodium chloride and water, and then dry filtered over anhydrous magnesium sulfate. The filtrate was concentrated under reduced pressure and purified by column chromatography to give the title compound (41.19 g, 87%).
MS [M+H] = 575 (M+1)

### Step B: Preparation of (2S,4S)-1-((3S,4R)-1-(tert-butyl)-4-(4-chlorophenyl)pyrrolidine-3-carbonyl)-4-(N-((1S,4R)-4-methylcyclohexyl)isobuiyramido)pyrrolidine-2-carboxylic acid

Methyl (2*S*,4*S*)-1-((3*S*,4*R*)-1-(*tert*-butyl)-4-(4-chlorophenyl)pyrrolidine-3-carbonyl)-4-(*N-*((1*s*,4*R*)-4-methylcyclohexyl)isobutyramido)pyrrolidin-2-carboxylate (39.4 g, 68.62 mmol) obtained from step A above was dissolved in methanol (450 ml), followed by the addition of an aqueous solution of 6N sodium hydroxide (57.2 ml, 343.09 mmol). After stirring at room temperature for 16 hours and adjusting the pH to about 5 with aqueous 6N hydrochloric acid, the reaction solution was concentrated under reduced pressure. The concentrate was dissolved with dichloromethane, and the insoluble solid was filtered through a paper filter. The filtrate was concentrated under reduced pressure to give a crude (38.4 g, 99%), which was used in the next step without purification.
MS [M+H] = 561 (M+1)

### Step C: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide

(2*S*,4*S*)-1-((3*S*,4*R*)-1-(*tert*-butyl)-4-(4-chlorophenyll)pyrrolidine-3-carbonyl)-4-(*N*-((1*s*,4*R*)-4-methylcyclohexyl)isobutyramido)pyrrolidine-2-carboxylic acid (38.4 g, 68.60 mmol), 1-(3-dimethylaminopropyl)-3-ethylcarbodiimide hydrochloride (18.4 g, 96.04 mmol) and 1-hydroxybenzotriazole hydrate (13.0 g, 96.04 mmol) obtained from step B above were dissolved in *N*,*N'*-dimethylformamide (200 ml), followed by the sequential addition of morpholine (5.9 ml, 68.80 mmol) and *N*,*N*'-diisopropylethylamine (59.7 ml, 343.02 mmol) slowly. After stirring at room temperature for 16 h, the reaction solution was concentrated under reduced pressure; then 0.5 N aqueous sodium hydroxide solution was added and extracted twice with ethyl acetate. The organic layer was washed twice with aqueous sodium chloride and water, and then dried over anhydrous magnesium sulfate. The filtrate was concentrated under reduced pressure and purified by column chromatography to give the title compound (37.05 g, 86%).
MS [M+H] = 630 (M+1)

### Step D: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

*N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N-*((1*s*,4*R*)-4-methylcyclohexyl)isobutyramide hydrochloride (5.0 g, 7.95 mmol) obtained from the step C above was dissolved in ethyl acetate (50 ml), then a 2N ethyl acetate solution of hydrochloric acid (3.97 ml, 15.89 mmol) was added slowly. After stirring at room temperature for 30 min, the reaction solvent was concentrated under reduced pressure. The resulting crude solid was purified by trituration using hexane and diethyl ether to give the title compound (5.23 g, 99%).
MS [M+H] = 630 (M+1)
¹H NMR (500 MHz, CD₃OD) δ 7.49-7.44 (m, 4H), 4.83 (m, 1H), 4.23-4.20 (m, 1H), 3.95-3.91 (m, 2H), 3.79-3.47 (m, 14H), 3.03-3.00 (m, 1H), 2.86-2.82 (m, 1H), 2.73-2.67 (m, 1H), 2.20-2.14 (m, 1H), 1.97 (m, 1H), 1.80-1.62 (m, 5H), 1.50 (s, 9H), 1.44-1.27 (m, 3H), 1.06-1.04 (m, 9H)

### Working Example: Pome knock-out mice model experiments

Proopiomelanocortin (POMC) is a precursor polypeptide of alpha-MSH, a ligand for the melanocortin-4 receptor (MC4R), which acts upstream of the melanocortin-4 receptor; Pomc knock-out mice, in which this proopiomelanocortin protein is not properly expressed, are models that reflect the genetic obesity disease POMC deficiency obesity, in which mice exhibit abnormal metabolic disease-related phenotypes such as hyperphagia and severe obesity.

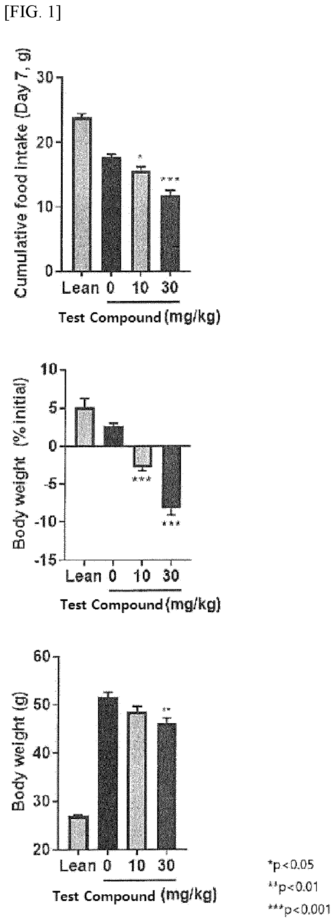

Pomc heterozygous knock-out mice, in which the Pomc gene encoding proopiomelanocortin (POMC) is not normally expressed, were fed a 60 kcal% high-fat diet (diet with 60 kcal% fat), while administering *N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N-*((1*s*,4*R*)-4-methylcyclohexyl)isobutyramide hydrochloride (hereinafter referred to as "test compound") obtained from the example of preparation at a dose of 10 mg/kg or 30 mg/kg for about 3 weeks to determine the anti-obesity efficacy, and the results are shown in FIG. 1.

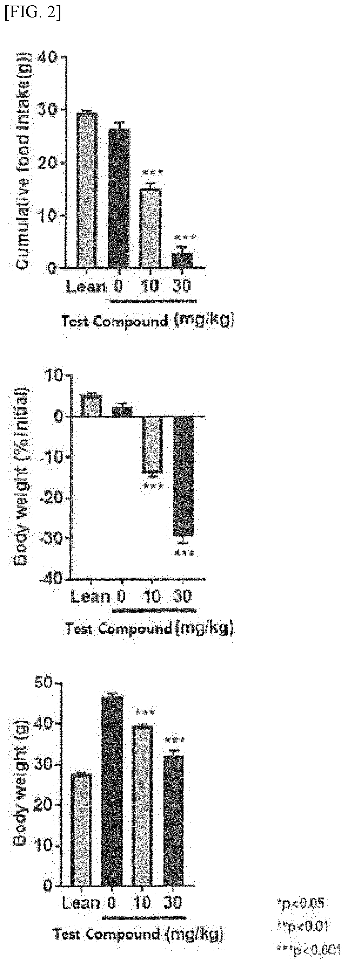

Pomc homozygous knock-out mice, in which the Pomc gene encoding proopiomelanocortin (POMC) is not expressed, were administered with the test compound at a dose of 10 mg/kg or 30 mg/kg for about 10 days while fed a 45 kcal% high-fat diet (diet with 45 kcal% fat) to determine the anti-obesity efficacy, and the results are shown in FIG. 2.

As can be seen from FIGs. 1 and 2, it can be confirmed that the test compound of the present invention exhibits excellent anti-obesity effects.

## Claims

1. A medicament for preventing or treating rare genetic obesity diseases associated with proopiomelanocortin (POMC) deficiency, comprising a therapeutically effective amount of a compound of Formula 1 or a pharmaceutically acceptable salt thereof: 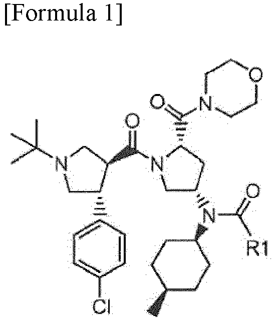 In the Formula 1, R1 is C₂-C₅ alkyl.

2. The medicament of claim 1, **characterized in that** the compound of the Formula 1 is *N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N-*((1*s*,4*R*)-4-methylcyclohexyl)isobutyramide of the following Formula 2:

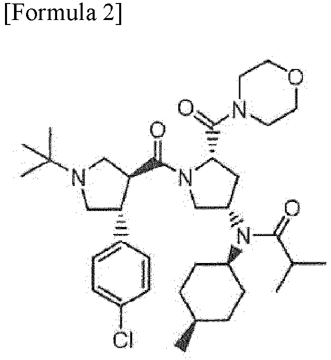

3. The medicament of claim 1, **characterized in that** the pharmaceutically acceptable salt is selected from the group consisting of hydrochloric acid, sulfuric acid, nitric acid, phosphoric acid, hydrobromic acid, and hydroiodic acid.

4. The medicament of claim 1, **characterized in that** it is an oral dosage form.

5. A pharmaceutical composition for preventing or treating rare genetic obesity diseases associated with proopiomelanocortin (POMC) deficiency, comprising a therapeutically effective amount of a compound of Formula 1 or a pharmaceutically acceptable salt thereof, together with a pharmaceutically acceptable carrier: 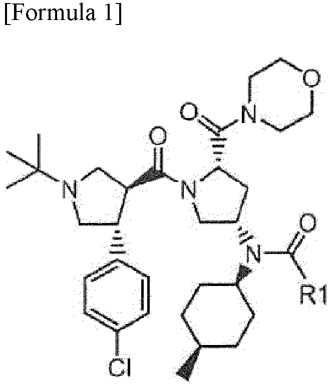 In the Formula 1, R1 is C₂-C₅ alkyl.

6. The pharmaceutical composition of claim 5, **characterized in that** the compound of Formula 1 is *N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N-*((1*s*,4*R*)-4-methylcyclohexyl)isobutyramide of Formula 2:

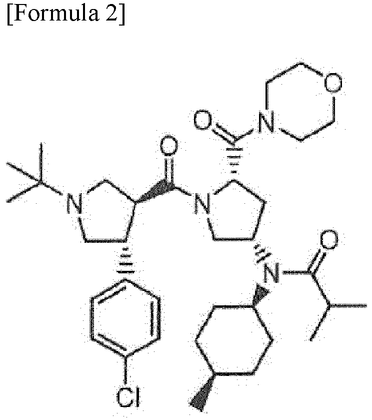

7. The pharmaceutical composition of claim 5, **characterized in that** the pharmaceutically acceptable salt is selected from the group consisting of hydrochloric acid, sulfuric acid, nitric acid, phosphoric acid, hydrobromic acid, and hydroiodic acid.

8. The pharmaceutical composition of claim 5, **characterized in that** it is an oral formulation.

9. Use of a compound of formula 1 or a pharmaceutically acceptable salt thereof for preparation of a medicament for preventing or treating rare genetic obesity diseases associated with proopiomelanocortin (POMC) deficiency: 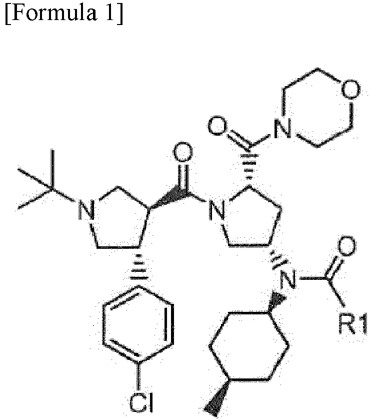 In the Formula 1, R1 is C₂-C₅ alkyl.

10. The use of claim 9, **characterized in that** the compound of Formula 1 is *N-*((3*S*,5*S*11.-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N-*((1*s*,4*R*)-4-methylcyclohexyl)isobutyramide of Formula 2:

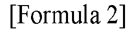

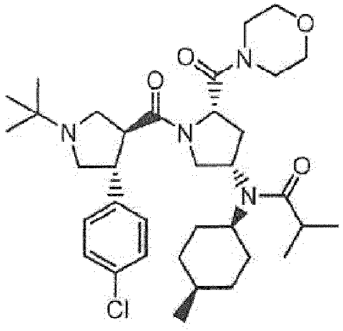

11. The use of claim 9, **characterized in that** the pharmaceutically acceptable salt is selected from the group consisting of hydrochloric acid, sulfuric acid, nitric acid, phosphoric acid, hydrobromic acid, and hydroiodic acid.

12. The use of claim 9, **characterized in that** the medicament is an oral formulation.
